Europäisches Patentamt

European Patent Office

Office européen des brevets

(11) Veröffentlichungsnummer: **0 086 745**
**B1**

# EUROPÄISCHE PATENTSCHRIFT

(45) Veröffentlichungstag der Patentschrift:
**14.11.84**

(21) Anmeldenummer: **83810049.3**

(22) Anmeldetag: **07.02.83**

(51) Int. Cl.³: **C 07 C 43/295**, C 07 C 41/34

(54) **Verfahren zur Herstellung einer sehr reinen Handelsform von 4,2',4'-Trichlor-2-hydroxydiphenyläther.**

(30) Priorität: **11.02.82 CH 853/82**

(43) Veröffentlichungstag der Anmeldung:
**24.08.83 Patentblatt 83/34**

(45) Bekanntmachung des Hinweises auf die Patenterteilung:
**14.11.84 Patentblatt 84/46**

(84) Benannte Vertragsstaaten:
**BE CH DE FR GB IT LI**

(56) Entgegenhaltungen:
**EP - A - 0 034 723**
**DE - B - 1 216 882**

(73) Patentinhaber: **CIBA-GEIGY AG, Postfach,
CH-4002 Basel (CH)**

(72) Erfinder: **Schreiber, Werner, Riehenstrasse 264,
CH-4058 Basel (CH)**
Erfinder: **Märky, Michael, Dr., Kurzelängeweg 1,
CH-4123 Allschwil (CH)**

# Beschreibung

Die vorliegende Erfindung betrifft ein Verfahren zur Herstellung einer sehr reinen, staubfreien und gerucharmen granulierten Handelsform von 4, 2', 4'-Trichlor-2-hydroxydiphenyläther.

Halogenierte 2-Hydroxydiphenyläther sind als gute antimikrobielle Wirkstoffe bekannt. Siehe z.B. US-Patentschriften Nrn. 3904696, 3629477 und 3800048 sowie Schweizer Patentschrift Nr. 432119. 4,2',4'-Trichlor-2-hydroxydiphenyläther ist auf Grund seiner hervorragenden Eigenschaften als bakterizider und fungizider Wirkstoff schon lange im Handel erhältlich. Er wird beispielsweise in Seifen, Waschmitteln, Kosmetika und anderen Haushaltartikeln als desinfizierender Wirkstoff eingesetzt. Auf Grund dieser Verwendung werden an die Reinheit des Wirkstoffes sehr hohe Anforderungen gestellt. Es ist daher besonders wichtig, dass 4,2',4'-Trichlor-2-hydroxydiphenyläther in möglichst reiner, d.h. möglichst wenig Nebenprodukte enthaltender Form hergestellt wird und dass diese Reinheit durch ein möglichst rationelles und technisch optimales Verfahren erreicht wird.

Mögliche Herstellungsverfahren für die erwähnte Verbindung sind in den oben zitierten Patentschriften beschrieben. Das einzige Herstellungsverfahren, das bisher grosstechnisch angewendet wurde, ist im Prinzip in der deutschen Auslegeschrift Nr. 1216882 beschrieben (s. besonders Beispiel 1). Dieses Verfahren wird folgendermassen ausgeführt: 4,2',4'-Trichlor-2-aminodiphenyläther wird in konzentrierter Schwefelsäure diazotiert und das erhaltene Diazoniumsalz, gegebenenfalls nach Zusatz von o-Dichlorbenzol, in etwa 50 bis 80 %iger Schwefelsäure verkocht. Zur Aufarbeitung wird das nach Abtrennen von der wässerigen Schicht erhaltene Gemisch aus 4,2',4'-Trichlor-2-hydroxydiphenyläther und 2,4,8-Trichlordibenzofuran (Nebenprodukt) stark alkalisch gemacht und gegebenenfalls vorhandenes organisches Lösungsmittel mit Wasserdampf abdestilliert. Das Nebenprodukt wird abfiltriert und im Filtrat der 4,2',4'-Trichlor-2-hydroxydiphenyläther durch Einstellung eines pH-Wertes von etwas über 9 ausgefällt. Das Produkt wird zur weiteren Reinigung im Vakuum destilliert und anschliessend aus Petroläther umkristallisiert.

Obwohl das chemische Herstellungsverfahren *via* Diazotierung und Verkochen eine grosse Menge des oben erwähnten Nebenproduktes liefert, scheint es technisch das einzige gangbare Verfahren zu sein. Das Problem besteht hauptsächlich darin, die Aufarbeitung so durchzuführen, dass möglichst wenig Verlust an Produkt in Kauf genommen werden muss, die Verunreinigungen auf das geforderte Mass reduziert werden, die Aufarbeitung technisch möglichst einfach und vor allem vom Standpunkt der Betriebssicherheit akzeptabel ist und das erhaltene Produkt in einer für den Handel brauchbaren Form anfällt.

Das bisher ausgeübte, oben beschriebene Verfahren, insbesondere die durchgeführte Aufarbeitung und Reinigung, hat einige Nachteile:

1) Um die geforderten Reinheitskriterien zu erfüllen, muss nach der Vakuumdestillation des Endproduktes ein (im grosstechnischen Massstab besonders aufwendiger) Umkristallisierungsschritt (aus Petroläther) eingeschaltet werden. Wegen der statischen Aufladung des Produktes stellt diese Umkristallisation ein beträchtliches Sicherheitsrisiko dar.

2) Bei der Vakuumdestillation, bei der das Produkt relativ hoch erhitzt werden muss, treten unerwünschte Reaktionen auf, die wieder zu unerwünschten Nebenprodukten führen und die die Endausbeute herabsetzen.

3) Das erhaltene Produkt enthält trotz der genannten aufwendigen Reinigungsoperationen noch einige Nebenprodukte, deren Eliminierung wünschenswert wäre.

4) Das Produkt fällt als Pulver an, das keine ideale Handelsform darstellt, da es zu Staubentwicklung und zu Zusammenballungen neigt.

5) Das Produkt weist einen charakteristischen Geruch auf, der nicht erwünscht ist.

Aufgabe der vorliegenden Erfindung war es, im oben geschilderten Basisverfahren die Aufarbeitung und Reinigung so zu gestalten, dass die beschriebenen Nachteile nicht oder vermindert auftreten und dass zusätzlich weitere technische Vorteile resultieren. Die Lösung dieser Aufgabe wurde überraschenderweise durch eine Kombination von besonderen Trennungs- und Reinigungsschritten erreicht. Die durch das erfindungsgemässe Verfahren erreichten Vorteile sind u.a.:

1) Es wird ein besonders reines Endprodukt erhalten, das die geforderten Reinheitskriterien voll erfüllt.

2) Es ist keine technisch fragwürdige Umkristallisation mehr nötig.

3) Die Endausbeute kann beträchtlich gesteigert werden.

4) Das Produkt fällt als staubfreies, gerucharmes, giessbares und daher leicht dosierbares Granulat an, das sich hervorragend als Handelsform eignet.

Das Verfahren zur Herstellung einer sehr reinen, staubfreien und gerucharmen granulierten Handelsform von 4,2',4'-Trichlor-2-hydroxydiphenyläther durch Diazotieren von 4,2',4'-Trichlor-2-aminodiphenyläther, Verkochen des erhaltenen Diazoniumsalzes und Aufarbeitung der 4,2',4'-Trichlor-2-hydroxydiphenyläther und 2,4,8-Trichlordibenzofuran als Reaktionsprodukte enthaltenden Reaktionsmischung, ist dadurch gekennzeichnet, dass man zur Aufarbeitung und Reinigung

a) die nach der Verkochung des Diazoniumsalzes und nach Abtrennung der wässerigen sauren Phase erhaltene, 4,2',4'-Trichlor-2-hydroxydiphenyläther und 2,4,8-Trichlordibenzofuran enthaltende Mischung stark alkalisch macht und

α) sie einer Wasserdampfdestillation zur Entfernung des organischen Lösungsmittels unterwirft, falls ein solches im Reaktionsgemisch vorhanden war, das ausgefallene 2,4,8-Trichlordibenzofuran abfiltriert und das Filtrat mehrmals mit einem inerten, mit Wasser nicht mischbaren organischen Lösungsmittel extrahiert, oder β) für den Fall, dass im Reaktionsgemisch kein oder nur wenig Lösungsmittel vohanden war, sie direkt mit einem inerten, mit Wasser nicht mischbaren organischen Lösungsmittel mehrmals extrahiert,

b) die stark alkalische wässerige Phase mit Säure auf einen pH-Wert von 3 bis 10 einstellt und den als Schmelze ausgefallenen 4,2',4'-Trichlor-2-hydroxydiphenyläther abtrennt,

c) aus dieser Schmelze, gegebenenfalls nach Zugabe eines schwerflüchtigen Verdünnungsmittels, leichtflüchtige Anteile entfernt und die verbleibende Schmelze anschliessend einer Kurzwegdestillation unterwirft,

d) die destillierte Schmelze in eine wässerige Dispersion von kristallinem 4,2',4'-Trichlor-2-hydroxydiphenyläther einleitet, wobei diese Dispersion gleichzeitig einer Nassmahlung unterzogen wird, und

e) das so erhaltene granulatartige Produkt abfiltriert und trocknet.

Die Diazotierung des 4,2',4'-Trichlor-2-aminodiphenyläthers sowie die Verkochung des Diazoniumsalzes zur entsprechenden Hydroxyverbindung kann nach üblichen, aus der Literatur bekannten Verfahren erfolgen, wie beispielsweise in den eingangs zitierten Patentschriften beschrieben. Vorteilhaft kann 4,2',4'-Trichlor-2-aminodiphenyläther mit Nitrosylschwefelsäure in Schwefelsäure diazotiert werden. Die Verkochung des Diazoniumsalzes kann dann z.B., gegebenenfalls nach Zusatz eines hochsiedenden Lösungsmittels (z.B. o-Dichlorbenzol), bei Temperaturen oberhalb 100° C erfolgen. Siehe etwa Beispiel 1 der deutschen Auslegeschrift Nr. 1216882.

Die erhaltene Reaktionsmischung ist stark sauer. Zunächst trennt man die saure wässerige Phase von der 4,2',4'-Trichlor-2-hydroxydiphenyläther und 2,4,8-Trichlordibenzofuran enthaltenden Mischung ab. Diese Mischung kann, falls zur Verkochung ein organisches Lösungsmittel zugegeben worden war, in letzterem gelöst sein. Im ersten erfindungsgemässen Schritt (Schritt a) wird zunächst diese Mischung stark alkalisch gemacht und in einer ersten Ausführungsform einer Wasserdampfdestillation unterworfen, um gegebenenfalls aus der Verkochung des Diazoniumsalzes vorhandenes Lösungsmittel (z.B. o-Dichlorbenzol) zu entfernen. Im Rückstand der Wasserdampfdestillation fällt das 2,4,8-Trichlordibenzofuran aus und wird abfiltriert. Das Filtrat wird mehrmals, vorzugsweise mindestens 3mal, z.B. 3- bis 10mal, insbesondere 3- bis 7mal mit einem inerten, mit Wasser nicht mischbaren, organischen Lösungsmittel extrahiert. Sofern im Reaktionsgemisch nach der Verkochung des Diazoniumsalzes und damit auch in der die Rohprodukte enthaltenden Mischung kein oder nur sehr wenig organisches Lösungsmittel enthalten ist, ist selbstverständlich eine Wasserdampfdestillation nicht nötig. In diesem Fall wird, nachdem die Mischung (Rohproduktgemisch in Form einer Schmelze) stark alkalisch gestellt wurde, das ausgefallene 2,4,8-Trichlordibenzofuran direkt abfiltriert und das Filtrat wie oben beschrieben extrahiert.

In einer zweiten Ausführungsform wird, sofern im Reaktionsgemisch nach der Verkochung kein oder nur wenig Lösungsmittel vorhanden war, ebenfalls die wässerige saure Phase von der 4,2',4'-Trichlor-2-hydroxydiphenyläther und 2,4,8-Trichlordibenzofuran enthaltenden Rohproduktmischung (Schmelze), die gegebenenfalls noch wenig Lösungsmittel enthalten kann, abgetrennt und letztere stark alkalisch gemacht. Dann wird die alkalische Schmelze (Rohproduktmischung) mit einem inerten, mit Wasser nicht mischbaren organischen Lösungsmittel mehrmals extrahiert. Auf diese Weise wird das Nebenprodukt 2,4,8-Trichlordibenzofuran zusammen mit weiteren Verunreinigungen weitgehend entfernt. Man extrahiert vorzugsweise mindestens 3mal, z.B. 3- bis 10mal, insbesondere 3- bis 7mal.

Die anfallenden Lösungsmittelextrakte werden vorzugsweise mit Natronlauge gewaschen, um gegebenenfalls in die Lösungsmittelphase gelangtes Endprodukt zurückzugewinnen. Die alkalische Waschlösung wird dann einem neuen Ansatz von noch nicht extrahierter, alkalischer Rohproduktlösung zugegeben.

Die oben beschriebene Abtrennung der 4,2',4'-Trichlor-2-hydroxydiphenyläther und 2,4,8-Trichlordibenzofuran enthaltenden Schmelze bzw. der diese enthaltenden organische Phase vom sauren wässerigen Reaktionsmedium erfolgt vorzugsweise bei erhöhter Temperatur, beispielsweise bei etwa 100 bis 120° C.

Wie bereits beschrieben, wird die abgetrennte Rohproduktmischung gemäss Stufe aα bzw. β stark alkalisch gemacht, und zwar derart, dass der 4,2',4'-Trichlor-2-hydroxydiphenyläther als Salz in Lösung geht. Vorzugsweise wird ein pH-Wert von ≧ 11 eingestellt. Die Einstellung dieses pH-Wertes kann beispielsweise mit einer wässerigen Lösung eines Alkalimetallhydroxids, insbesondere von Natriumhydroxid, vorgenommen werden.

Das gemäss Schritt a zur Extraktion verwendete Lösungsmittel muss gegenüber den Produkten, die in der Mischung enthalten sind, inert sein, darf nicht mit Wasser mischbar sein und soll nicht durch das alkalische Medium angegriffen werden. Als derartige Lösungsmittel kommen beispielsweise aromatische oder aliphatische Kohlenwasserstoffe, halogenierte aromatische oder aliphatische Kohlenwasserstoffe, mit Wasser nicht mischbare Äther, Ketone, u.a. in Betracht, insbesondere aber aromatische oder aliphatische Kohlenwasserstoffe oder halogenierte aromatische oder aliphatische Kohlenwasserstoffe. Bevorzugt sind aromatische Kohlenwasserstoffe oder halogenierte (z.B. chlorierte) aromatische oder aliphatische Kohlenwasserstoffe. Beispiele für derartige Lösungsmittel sind Methylenchlorid, Chloroform, Tetrachlorkohlenstoff, Äthylenchlorid, Tetrachloräthan, 1,2-Dichloräthan, 1,1,1-Trichloräthan, Perchloräthylen, Trichloräthylen, 1,1,2-Trichlortrifluoräthan, Dichlorbenzole wie 1,2-Dichlorbenzol, Trichlorbenzole, Toluol und Xylole. Besonders vorteilhaft verwendet man Dichlorbenzole, z.B. 1,2-Dichlorbenzol, Toluol oder Xylol (z.B. Xylol/Isomeren-Gemisch).

Die Extraktionen gemäss Schritt a werden vorzugsweise in einem Temperaturbereich zwischen Raumtemperatur und etwa 95° C durchgeführt. Besonders günstig haben sich Temperaturen zwischen 45 und 85° C erwiesen. Die Extraktionstemperatur hängt selbstverständlich weitgehend vom verwendeten Lösungsmittel ab.

Die nach der Extraktion erhaltene, gereinigte, stark alkalische wässerige Phase wird nun gemäss Schritt b mit einer beliebige Säure auf einen pH-Wert von 3 bis 10 eingestellt, um den 4,2′,4′-Trichlor-2-hydroxydiphenyläther auszufällen. Das als Schmelze anfallende Produkt wird abgetrennt. Vorzugsweise erfolgt die Ausfällung bei einem pH-Wert von 8 bis 10, insbesondere von 9 bis 9,5.

Aus der nach Schritt b erhaltenen Produktschmelze werden zunächst noch vorhandene leichtflüchtige Verunreinigungen, wie Lösungsmittelreste u.a., entfernt. Dies kann in einer konventionellen Destillationsapparatur geschehen, vorzugsweise in einem Fallfilmverdampfer. Alternativ kann man die leichtflüchtigen Anteile auch direkt in der Kurzwegdestillationsapparatur in einer zusätzlichen Destillationsstufe entfernen.

Vor des Destillation wird der Schmelze vorzugsweise noch eine geringe Menge eines schwerflüchtigen Verdünnungsmittels, z.B. eines Polyäthylenglykols, zugegeben, um auf diese Weise den Destillationsrückstand flüssig zu halten. Dadurch wird die Reinigung der Destillationsapparatur wesentlich erleichtert.

Nach Entfernung der leicht flüchtigen Anteile wird gemäss Schritt c die Produktschmelze einer Kurzwegdestillation unterworfen. Zu diesem Zweck verwendet man allgemein bekannte Kurzwegdestillationsapparaturen (Kurzwegverdampfer), wie sie in der Literatur beschrieben sind (s. z.B. „Ullmanns Encyklopädie der technischen Chemie", 4. Aufl., Bd. 2, S. 657 und 658). Die Kurzwegdestillation wird in der Literatur auch als Molekulardestillation und die entsprechenden Apparaturen werden auch als Molekularverdampfer bezeichnet.

Für die Reinigung von 4,2′,4′-Trichlor-2-hydroxydiphenyläther hat die Kurzwegdestillation den besonderen Vorteil, dass bei der Destillation keine sehr hohen Temperaturen angewendet werden müssen und dass das Produkt nur sehr kurze Zeit höheren Temperaturen ausgesetzt ist (bei der Kurzwegdestillation liegen die Destillationswege im Grössenordungsbereich der mittleren freien Weglänge der Moleküle). Auf diese Weise wird die Bildung von Zersetzungs- und Kondensationsprodukten (z.B. Dibenzodioxinen), wie sie bei konventioneller Destillation unerwünschterweise auftreten, weitgehend vermieden. Um die gennanten Vorteile optimal zu nützen, destilliert man vorzugsweise bei sehr niederen Drücken. Drücke in der Grössenordung von etwa $10^{-3}$ mbar werden bevorzugt angewendet.

Obwohl an sich eine Destillation zur Erreichung der geforderten Reinheitskriterien genügen würde, wird die Produktschmelze vorzugsweise zweimal destilliert. Man erhält dann ein noch reineres Endprodukt und das nach der ersten Destillation gegebenenfalls noch eine geringe gelbliche Färbung aufweisende Destillat wird praktisch farblos.

Wegen des relativ niedrigen Schmelzpunktes des 4,2′,4′-Trichlor-2-hydroxydiphenyläthers (55 bis 60° C) bereitete in den bisher angewandten Verfahren die Kristallisation der Schmelze gewisse Schwierigkeiten. Ausserdem war das erhaltene Kristallpulver als Handelsform nicht optimal geeignet, da es nicht staubfrei war und auch zu Klumpenbildung neigte. Im erfindungsgemässen Schritt d wird das nach Schritt c erhaltene Destillat gleichzeitig kristallisiert und zu einer granulierten Handelsform verarbeitet. Zu diesem Zweck wird das Destillat in eine wässerige Dispersion von kristallinem 4,2′,4′-Trichlor-2-hydroxydiphenyläther eingeleitet, wobei die Dispersion gleichzeitig einer Nassmahlung unterzogen wird.

Der Kristallisations- und Granulierungsschritt d wird vorzugsweise bei Temperaturen von 10 bis 50° C, insbesondere von 30 bis 45° C durchgeführt. Die Dispersion, in die das destillierte Produkt eingeleitet wird, kann sehr geringe Mengen an kristallinem 4,2′,4′-Trichlor-2-hydroxydiphenyläther enthalten. Bei sehr geringen Mengen erfolgt die Zugabe des Produktdestillats zweckmässig langsam, um eine Kristallisation zu erreichen. Vorzugsweise enthält die wässerige Dispersion beim Beginn der Zudosierung des Produktdestillats mindestens etwa 0,01, insbesondere mindestens etwa 0,1, z.B. mindestens etwa 0,5 Gew.-% an kristallinem 4,2′,4′-Trichlor-2-hydroxydiphenyläther. Durch Zugabe des zu kristallisierenden Produktes steigt der Anteil an kristallisiertem Produkt in der Dispersion an, und zwar in der Praxis so lange letztere noch gut rührbar ist. Beispielsweise bei etwa 30 bis 40 % Gehalt an kristallisiertem Produkt in der Dispersion wird die Zufuhr von weiterer Produktschmelze unterbrochen und der kristalline, granulatförmige 4,2′,4′-Trichlor-2-hydroxydiphenyläther abfiltriert.

Da die Dispersion während des Kristallisationsschrittes nass gemahlen wird, erfolgt letzterer zweckmässig in einem üblichen Nassmahlgerät. Entsprechende Geräte sind in grosser Zahl aus der Literatur bekannt und im Handel erhältlich. Die Nassmahlung kann z.B. in einer üblichen Kugelmühle erfolgen; bevorzugt wird die Nassmahlung in einer Kolloidmühle, insbesondere einer solchen auf dem Rotor-Stator-Prinzip bzw. in einer Zahnkolloidmühle durchgeführt.

Das nach Schritt d erhaltene Granulat wird filtriert und in üblicher Weise getrocknet. Das dann erhaltene, trockene, rein weisse Granulat ist rieselfähig, neigt nicht zur Agglomeration und ist praktisch geruchlos.

Die nachfolgenden Beispiele dienen der weiteren Erläuterung des erfindungsgemässen Verfahrens. Teile und Prozente sind darin, ebenso wie in der übrigen Beschreibung und in den Patentansprüchen, als Gewichtsteile und Gewichtsprozent zu verstehen, sofern nichts anderes angegeben ist.

*Beispiel 1 :*

92 g Nitrosylschwefelsäure (100 %ig) werden in 720 g konzentrierter Schwefelsäure gelöst und 200 g 4,2',4'-Trichlor-2-aminodiphenyläther unter gutem Rühren bei 50 bis 55° C innerhalb von 2 h eingetragen. Man rührt das Gemisch noch 3 h bei Raumtemperatur. Dann lässt man unter Kühlen mit Eiswasser 210 ml Wasser zulaufen, wobei die Temperatur auf 70° C steigt. Nach dem Zusatz von 370 ml o-Dichlorbenzol wird das Gemisch in einem Ölbad von 200° C so lange zum Sieden erhitzt, bis keine Diazoverbindung mehr nachweisbar ist. Dann wird die obere organische Schicht noch heiss abgetrennt, mit 460 ml Wasser versetzt und dann mit 30 %iger Natronlauge auf einen pH-Wert von $\geq$ 11 eingestellt. Danach wird das Gemisch einer Wasserdampfdestillation unterworfen, um o-Dichlorbenzol zu entfernen. Das im wässerigen Rückstand ausfallende 2,4,8-Trichlordibenzofuran wird abfiltriert und gewaschen.

Die vereinigten Filtrate werden in einen Kolben gebracht, mit 50 ml 1,2-Dichlorbenzol versetzt und auf 50° C erhitzt. Es wird 10 min stark gerührt. Nach Abstellen des Rührers trennen sich während 20 bis 30 min die Schichten. Die untere 1,2-Dichlorbenzolphase wird abgetrennt und die wässerige Schicht wieder mit 50 ml 1,2-Dichlorbenzol versetzt und bei 50° C gerührt. Diese Extraktionsoperation wird viermal wiederholt. Anschliessend wird die wässerige Phase mit 65 %iger Schwefelsäure auf pH 9 bis 9,5 eingestellt. Die entstandene Emulsion wird noch 30 min gerührt, der pH-Wert nochmals kontrolliert, der Rührer abgestellt und die sich abscheidende ölige Schmelze des 4,2',4'-Trichlor-2-hydroxydiphenyläthers abgetrennt.

Die abgetrennte Schmelze wird mit 3 g Polyäthylenglykol 600 versetzt. Anschliessend werden die noch vorhandenen leichtflüchtigen Anteile in einem Fallfilmverdampfer bei 100° C und 15 bis 30 mbar entfernt. Das entgaste Rohprodukt wird nun in einem Kurzwegverdampfer (Molekularverdampfer) bei einem Druck von etwa 10 mbar destilliert (Manteltemperatur ca 140 bis 160° C), wobei die schwerflüchtigen Verunreinigungen zusammen mit dem Polyäthylenglykol zurückbleiben. Das erhaltene, bereits reine, jedoch noch etwas gelbliche Destillat wird nochmals destilliert (Manteltemperatur 120 bis 130° C). Der Rückstand dieser Reinigungsstufe wird bei der nachfolgenden Destillation von weiterem 4,2',4'-Trichlor-2-hydroxydiphenyläther wieder zugesetzt.

Die destillierte Schmelze wird nun in einem heizbaren Tropftrichter vorgelegt und einer Dispersion von 5 g kristallinem 4,2',4'-Trichlor-2-hydroxydiphenyläther in 300 ml deionisiertem Wasser bei einer Temperatur von 35 bis 45° C zudosiert. Dabei wird die Dispersion in einer Kolloidmühle auf dem Rotor-Stator-Prinzip (z.B. Ultra-Turax", Polytron") intensiv gerührt. Mit Hilfe der Rührgeschwindigkeit kann man die Teilchengrösse des schliesslich erhaltenen Granulats bestimmen: Bei schnellerem Rühren erhält man ein feineres, bei langsamerem Rühren ein gröberes Granulat. Das erhaltene Granulat wird abfiltriert und getrocknet. Es zeigt kaum Staubenwicklung, ist praktisch geruchlos und bildet auch bei längerer Lagerung keine Klumpen.

Gemäss gaschromatographischer Analyse enthält das so erhaltene Produkt mehr als 99,5 % 4,2',4'-Trichlor-2-hydroxydiphenyläther, weniger als 1 ppm 2,4,8-Trichlordibenzofuran und weniger als 2 ppm 2,8-Dichlor-p-dibenzodioxin. Es enthält auch keine störenden Geruchskomponenten mehr.

Wiederholt man obiges Verfahren, setzt jedoch im Extraktionsschritt an Stelle von 1,2-Dichlorbenzol 1,2-Dichloräthan, 1,1,2,2-Tetrachloräthylen, Tetrachlorkohlenstoff, 1,1,2-Trichlortrifluoräthan, Toluol, o-Xylol, m-Xylol, p-Xylol oder Xylol/Isomeren-Gemisch als Lösungsmittel ein, so kommt man ebenfalls zu einem wie oben definierten reinen Endprodukt.

*Beispiel 2 :*

92 g Nitrosylschwefelsäure (100 %ig) werden in 720 g konzentrierter Schwelfelsäure gelöst und 200 g 4,2',4'-Trichlor-2-aminodiphenyläther unter gutem Rühren bei 40 bis 45° C innerhalb von 2 h eingetragen. Man rührt das Gemisch noch 3 h bei Raumtemperatur. Dann lässt man unter Kühlen mit Eiswasser 210 ml Wasser zulaufen, wobei die Temperatur auf 70° C steigt. Danach wird das Gemisch in einem Ölbad von 200° C so lange zum Sieden erhitzt, bis keine Diazoverbindung mehr nachweisbar ist. Das zweiphasige Reaktionsgemisch wird dann auf eine Temperatur von 120° C gebracht und die 4,2',4'-Trichlor-2-hydroxydiphenyläther und 2,4,8-Trichlordibenzofuran enthaltende Schmelze vom wässerigen schwelfelsauren Reaktionsmedium abgetrennt.

In einen Doppelmantel-Planschliffkolben mit Untenauslauf, Ankerrührer, Thermometer, pH-Meter und Rückflusskühler werden 83 ml 30 %ige Natronlauge, 720 ml Wasser und 230 ml Toluol vorgelegt und auf 80 bis 83° C erwärmt. Unter starkem Rühren wird die wie vorstehend abgetrennte Produktschmelze zugegeben. Der pH-Wert des zweiphasigen Gemisches wird kontrolliert und, falls nötig, mit Natronlauge auf den Sollwert von $\geq$ 11 eingestellt. Das Gemisch wird 15 min gerührt, der pH-Wert nochmals kontrolliert, dann der Rührer abgestellt und die wässerige Phase nach 15 min in einen Doppelmantel-Planschliffkolben abgetrennt und auf 50 bis 60° C abgekühlt. Die wässerige, alkalische Lösung wird nochmals mit 50 ml Toluol versetzt und 10 min gut gerührt. 20 bis 30 min nach Abstellen des Rührers wird die untere wässerige Phase abgetrennt. Dieser Extraktionsprozess wird insgesamt 5mal wiederholt. Die angefallenen Toluolphasen werden vereinigt und bei 50° C 3 bis 5mal mit 100 ml 1N NaOH extrahiert. Dieser wässerige Extrakt wird einem neuen Ansatz von nocht nicht extrahierter, alkalischer Rohproduktlösung zugefügt.

Die extrahierte wässerige Phase wird nun mit Schwefelsäure 65 % auf einen pH-Wert von 9,2 bis 9,5 gestellt und die Temperatur unter 60° C gehalten. Die erhaltene Emulsion wird 30 min bei 50 bis 55° C verrührt, der pH-Wert nochmals kontrolliert, und, falls nötig, korrigiert. Dann wird der Rührer abgestellt und die sich abscheidende, ölige Schmelze des 4,2',4'-Trichlor-2-hydroxydiphenyläthers etwa nach 30 min abgetrennt.

Die abgetrennte Schmelze wird mit 3 g Polyäthylenglykol 600 versetzt. Anschliessend werden die noch vorhandenen leichtflüchtigen Anteile in einem Fallfilmverdampfer bei 100°C und 15 bis 30 mbar entfernt. Das entgaste Rohprodukt wird nun in einem Kurzwegverdampfer (Molekularverdampfer) bei einem Druck von etwa $10^{-3}$ mbar destilliert (Manteltemperatur ca. 140 bis 160°C), wobei die schwerflüchtigen Verunreinigungen zusammen mit dem Polyäthylenglykol zurückbleiben. Das erhaltene, bereits reine, jedoch noch etwas gelbliche Destillat wird nochmals destilliert (Manteltemperatur 120 bis 130°C). Der Rückstand dieser Reinigungsstufe wird bei der nachfolgenden Destillation von weiterem rohem 4,2',4'-Trichlor-2-hydroxydiphenyläther wieder zugesetzt.

Die destillierte Schmelze wird nun in einem heizbaren Tropftrichter vorgelegt und einer Dispersion von 5 g kristallinem 4,2',4'-Trichlor-2-hydroxydiphenyläther in 300 ml deionisiertem Wasser bei einer Temperatur von 35 bis 45°C zudosiert. Dabei wird die Dispersion in einer Kolloidmühle auf dem Rotor-Stator-Prinzip (z.B. Ultra-Turax", Polytron") intensiv gerührt. Mit Hilfe der Rührgeschwindigkeit kann man die Teilchengrösse des schliesslich erhaltenen Granulats bestimmen: Bei schnellerem Rühren erhält man ein feineres, bei langsamerem Rühren ein gröberes Granulat. Das erhaltene Granulat wird abfiltriert und getrocknet. Es zeigt kaum Staubentwicklung, ist praktisch geruchlos und bildet auch bei längerer Lagerung kleine Klumpen.

Gemäss gaschromatographischer Analyse enthält das so erhaltene Produkt mehr als 99,5 % 4,2',4'-Trichlor-2-hydroxydiphenyläther, weniger als 1 ppm 2,4,8-Trichlordibenzofuran und weniger als 2 ppm 2,8-Dichlor-p-dibenzodioxin. Es enthält auch keine störenden Geruchskomponenten mehr.

Wiederholt man vorstehendes Verfahren, setzt jedoch zur Extraktion der Schmelze an Stelle von Toluol andere aromatische Kohlenwasserstoffe, z.B. Xylol, als Lösungsmittel ein, so kommt man ebenfalls zu einem wie vorstehend definierten reinen Endprodukt.

Die Extraktion kann aber auch beispielsweise mit 1,2-Dichlorbenzol, 1,1,2,2-Tetrachloräthan, Tetrachloräthylen, Chloroform oder 1,1,2-Trichlortrifluoräthan durchgeführt werden.

### Patentansprüche

1. Verfahren zur Herstellung von 4,2',4'-Trichlor-2-hydroxydiphenyläther als sehr reine, staubfreie und gerucharme granulierte Handelsform durch Diazotieren von 4,2',4'-Trichlor-2-aminodiphenyläther, Verkochen des erhaltenen Diazoniumsalzes und Aufarbeitung der 4,2',4'-Trichlor-2-hydroxydiphenyläther und 2,4,8-Trichlordibenzofuran als Reaktionsprodukte enthaltenden Reaktionsmischung, dadurch gekennzeichnet, dass man zur Aufarbeitung und Reinigung

a) die nach der Verkochung des Diazoniumsalzes und nach Abtrennung der wässerigen sauren Phase erhaltene, 4,2',4'-Trichlor-2-hydroxydiphenyläther und 2,4,8-Trichlordibenzofuran enthaltende Mischung stark alkalisch macht, α) sie einer Wasserdampfdestillation zur Entfernung des organischen Lösungsmittels unterwirft, falls ein solches im Reaktionsgemisch vorhanden war, das ausgefallene 2,4,8-Trichlordibenzofuran abfiltriert und das Filtrat mehrmals mit einem inerten, mit Wasser nicht mischbaren organischen Lösungsmittel extrahiert, oder β) für den Fall, das im Reaktionsgemisch kein oder nur wenig Lösungsmittel vorhanden war, sie direkt mit einem inerten, mit Wasser nicht mischbaren organischen Lösungsmittel mehrmals extrahiert,

b) die stark alkalische wässerige Phase mit Säure auf einen pH-Wert von 3 bis 10 einstellt und den als Schmelze ausgefallenen 4,2',4'-Trichlor-2-hydroxydiphenyläther abtrennt,

c) aus dieser Schmelze, gegebenenfalls nach Zugabe eines schwerflüchtigen Verdünnungsmittels, leichtflüchtige Anteile entfernt und die verbleibende Schmelze anschliessend einer Kurzwegdestillation unterwirft,

d) die destillierte Schmelze in eine wässerige Dispersion von kristallinem 4,2',4'-Trichlor-2-hydroxydiphenyläther einleitet, wobei diese Dispersion gleichzeitig einer Nassmahlung unterzogen wird, und

e) das so erhaltene granulatartige Produkt abfiltriert und trocknet.

2. Verfahren nach Anspruch 1, dadurch gekennzeichnet, dass die 4,2',4'-Trichlor-2-hydroxydiphenyläther und 2,4,8-Trichlordibenzofuran enthaltende Mischung gemäss Schritt (a) auf einen pH-Wert $\geq$ 11 gebracht wird.

3. Verfahren nach einem der beiden Ansprüche 1 oder 2, dadurch gekennzeichnet, dass man für die Extraktion gemäss Schritt (a) einen aliphatischen oder aromatischen Kohlenwasserstoff oder einen halogenierten, vorzugsweise chlorierten, aliphatischen oder aromatischen Kohlenwasserstoff einsetzt.

4. Verfahren nach Anspruch 3, dadurch gekennzeichnet, dass man für die Extraktion einen aromatischen Kohlenwasserstoff oder einen halogenierten, vorzugsweise chlorierten, aliphatischen oder aromatischen Kohlenwasserstoff verwendet.

5. Verfahren nach Anspruch 4, dadurch gekennzeichnet, dass man für die Extraktion Methylenchlorid, Chloroform, Tetrachlorkohlenstoff, Äthylenchlorid, Tetrachloräthan, 1,2-Dichloräthan, 1,1,1-Trichloräthan, Perchloräthylen, Trichloräthylen, 1,1,2-Trichlortrifluoräthan, Dichlorbenzole, Trichlorbenzole, Toluol oder Xylole, vorzugsweise 1,2-Dichlorbenzol, Toluol oder Xylol verwendet.

6. Verfahren nach einem der Ansprüche 1 bis 5, dadurch gekennzeichnet, dass man die Extraktion gemäss Schritt (a) 3- bis 10mal, vorzugsweise 3- bis 7mal, durchführt.

7. Verfahren nach einem der Ansprüche 1 bis 6, dadurch gekennzeichnet, dass die Extraktionen gemäss Schritt (a) bei Temperaturen zwischen Raumtemperatur und 95° C durchgeführt werden.

8. Verfahren nach Anspruch 7, dadurch gekennzeichnet, dass man bei Temperaturen von 45 bis 85° C extrahiert.

9. Verfahren nach Anspruch 1, dadurch gekennzeichnet, dass man gemäss Schritt (b) die wässerige Phase auf einen pH-Wert von 8 bis 10, vorzugsweise von 9 bis 9,5, einstellt.

10. Verfahren nach Anspruch 1, dadurch gekennzeichnet, dass man der Schmelze vor der Destillation gemäss Schritt (c) Polyäthylenglykol zugibt.

11. Verfahren nach Anspruch 1, dadurch gekennzeichnet, dass man die leichtflüchtigen Anteile der Schmelze vor der Kurzwegdestillation in einem Fallfilmverdampfer entfernt.

12. Verfahren nach Anspruch 1, dadurch gekennzeichnet, dass man die leichtflüchtigen Anteile der Schmelze direkt in der Kurzwegdestillationsapparatur in einer zusätzlichen Destillationsstufe entfernt.

13. Verfahren nach Anspruch 1, dadurch gekennzeichnet, dass man die Kurzwegdestillation bei sehr niedrigen Drucken, vorzugsweise in der Grössenordnung von $10^{-3}$ mbar, durchführt.

14. Verfahren nach Anspruch 1, dadurch gekennzeichnet, dass man gemäss Schritt (c) die Schmelze zweimal in der Kurzwegdestillationsapparatur destilliert.

15. Verfahren nach Anspruch 1, dadurch gekennzeichnet, dass die Einleitung der Schmelze gemäss Schritt (d) in die wässerige Dispersion von kristallinem 4,2',4'-Trichlor-2-hydroxydiphenyläther bei Temperaturen von 10 bis 50° C, insbesondere von 30 bis 45° C, erfolgt.

16. Verfahren nach Anspruch 1, dadurch gekennzeichnet, dass die wässerige Dispersion gemäss Schritt (d) mindestens 0,1, insbesondere mindestens 0,5 %, an kristallinem 4,2',4'-Trichlor-2-hydroxydiphenyläther enthält.

17. Verfahren nach Anspruch 1, dadurch gekennzeichnet, dass Schritt (d) in einer Kolloidmühle auf dem Rotor-Stator-Prinzip durchgeführt wird.

18. Verfahren nach Anspruch 1, dadurch gekennzeichnet, dass für den Fall, dass im Reaktionsgemisch kein oder nur wenig Lösungsmittel vorhanden war, Schritt (aα) ohne Wasserdampfdestillation durchgeführt wird.

## Claims

1. A process for the preparation of 4,2',4'-trichloro-2-hydroxydiphenyl ether as very pure, non-dusting, substantially odourless granular physical form by diazotising 4,2',4'-trichloro-2-aminodiphenyl ether, boiling the resulting diazonium salt and working up the reaction mixture containing the reaction products 4,2',4'-trichloro-2-hydroxydiphenyl ether and 2,4,8-trichlorodibenzofuran, wherein working up and purification comprises,

(*a*) making the mixture which is obtained after boiling the diazonium salt and separating off the aqueous acid phase and which contains 4,2',4'-trichloro-2-hydroxydiphenyl ether and 2,4,8-trichlorodibenzofuran strongly alkaline, (α) subjecting it to steam distillation to remove the organic solvent, if such a solvent was present in the reaction mixture, isolating the precipitated 2,4,8-trichlorodibenzofuran by filtration and extracting the filtrate repeatedly with an inert water-immiscible organic solvent, or (β) if only little or no solvent was present in the reaction mixture, extracting the alkaline mixture direct repeatedly with an inert water-immiscible organic solvent,

(*b*) adjusting the pH value of the strongly alkaline aqueous phase to 3 to 10 with an acid and isolating the 4,2',4'-trichloro-2-hydroxydiphenyl ether obtained as a melt,

(*c*) removing highly volatile constituents from said melt, if desired after addition of a diluent of low volatility, and then subjecting the residual melt to molecular distillation,

(*d*) introducing the distilled melt into an aqueous dispersion of crystalline 4,2',4'-trichloro-2-hydroxydiphenyl ether, while simultaneously subjecting this dispersion to wet grinding, and isolating the resulting granular product by filtration and drying it.

2. A process according to Claim 1, wherein the mixture containing 4,2',4'-trichloro-2-hydroxydiphenyl ether and 2,4,8-trichlorodibenzofuran is brought to a pH value $\geq$ 11 according to step (*a*).

3. A process according to Claim 1 or 2, wherein an aliphatic or aromatic hydrocarbon or a halogenated, preferably chlorinated, aliphatic or aromatic hydrocarbon is used for the extraction according to step (*a*).

4. A process according to Claim 3, wherein an aromatic hydrocarbon or a halogenated, preferably chlorinated, aliphatic or aromatic hydrocarbon is used for the extraction.

5. A process according to Claim 4, wherein methylene chloride, chloroform, carbon tetrachloride, ethylene chloride, tetrachloroethane, 1,2-dichloroethane, 1,1,1-trichloroethane, perchloroethylene, trichloroethylene, 1,1,2-trichlorotrifluoroethane, a dichlorobenzene, a trichlorobenzene, toluene or a xylene, preferably 1,2-dichlorobenzene, toluene or xylene, is used for the extraction.

6. A process according to any one of Claims 1 to 5, wherein the extraction according to step (*a*) is carried out 3 to 10 times, preferably 3 to 7 times.

7. A process according to any one of Claims 1 to 6, wherein the extraction according to step (*a*) is carried out in the temperature range from room temperature to 95° C.

8. A process according to Claim 7, wherein the extraction is carried out in the temperature range from 45 to 85° C.

9. A process according to Claim 1, wherein the aqueous phase is adjusted to a pH value of 8 to 10, preferably 9 to 9.5, according to step (*b*).

10. A process according to Claim 1, wherein polyethylene glycol is added to the melt before the distillation according to step (*c*).

11. A process according to Claim 1, wherein the highly volatile constituents are removed from the melt in a falling film evaporator before the molecular distillation.

12. A process according to Claim 1, wherein the highly volatile constituents are removed from the melt direct in an additional distillation stage in the molecular distillation apparatus.

13. A process according to Claim 1, wherein the molecular distillation is carried out under very low pressures, preferably of the order of $10^{-3}$ mbar.

14. A process according to Claim 1, wherein the melt is distilled twice in the molecular distillation apparatus according to step (*c*).

15. A process according to Claim 1, wherein the melt is introduced, according to step (*d*), into the aqueous dispersion of crystalline 4,2',4'-trichloro-2-hydroxydiphenyl ether at temperatures of 10 to 50° C, in particular 30 to 45° C.

16. A process according to Claim 1, wherein the aqueous dispersion according to step (*d*) contains at least 0.1 %, in particular at least 0.5 %, of crystalline 4,2',4'-trichloro-2-hydroxydiphenyl ether.

17. A process according to Claim 1, wherein step (*d*) is carried out in a colloid mill based on the rotor-stator principle.

18. A process according to Claim 1, wherein step (aα) is carried out without steam distillation, if only little or no solvent was present in the reaction mixture.

**Revendications**

1. Procédé pour la préparation de l'éther 4,2',4'-trichloro-2-hydroxydiphényle sous forme commerciale granulée très pure, sans poussière et peu odorant, par diazotation de l'éther 4,2',4'-trichloro-2-aminodiphényle, transformation par ébullition du sel de diazonium obtenu et traitement du mélange réactionnel contenant l'éther 4,2',4'-trichloro-2-hydroxydiphényle et le 2,4,8-trichlorodibenzofuranne comme produits de réactions, caractérisé par le fait que, pour le traitement et la purification,

a) on rend très fortement alcalin le mélange contenant l'éther 4,2',4'-trichloro-2-hydroxydiphényle et le 2,4,8-trichlorodibenzofuranne, obtenu après la transformation par ébullition du sel de diazonium et, après séparation de la phase acide aqueuse, α) on soumet ce mélange à un entraînement à la vapeur d'eau pour éliminer le solvant organique au cas où un tel solvant est présent dans le mélange réactionnel, on sépare par filtration le 2,4,8-trichlorodibenzofuranne précipité et on extrait le filtrat plusieurs fois avec un solvant organique inerte non miscible avec l'eau ou, β) dans le cas où il n'y a pas de solvant ou seulement qu'une très faible quantité de celui-ci dans le mélange réactionnel, on extrait directement ce dernier plusieurs fois avec un solvant organique inerte non miscible avec l'eau,

b) on règle le pH de la phase aqueuse fortement alcaline à 3 à 10 avec un acide et on sépare l'éther 4,2',4'-trichloro-2-hydroxydiphényle précipité sous forme d'une masse fondue,

c) à partir de cette masse fondue, on élimine les parties très volatiles, éventuellement après avoir ajouté un diluant très peu volatil, et on soumet la masse fondue restante à une distillation moléculaire,

d) on introduit la masse fondue distillée dans une dispersion aqueuse d'éther 4,2',4'-trichloro-2-hydroxydiphényle cristallisé, cette dispersion étant soumise simultanément à un broyage à l'état humide, et

e) on sépare par filtration et on sèche le produit en granulés ainsi obtenu.

2. Procédé selon la revendication 1, caractérisé par le fait que le mélange contenant l'éther 4,2',4'-trichloro-2-hydroxydiphényle et le 2,4,8-trichlorodibenzofuranne, selon le stade (a) est amené à un pH $\geq$ 11.

3. Procédé selon l'une des revendications 1 ou 2, caractérisé par le fait qu'on utilise, pour l'extraction selon le stade (a), un hydrocarbure aliphatique ou aromatique ou un hydrocarbure aliphatique ou aromatique halogéné, de préférence chloré.

4. Procédé selon la revendication 3, caractérisé par le fait qu'on utilise pour l'extraction un hydrocarbure aromatique ou un hydrocarbure aliphatique ou aromatique halogéné, de préférence chloré.

5. Procédé selon la revendication 4, caractérisé par le fait qu'on utilise pour l'extraction le chlorure de méthylène, le chloroforme, le tétrachlorure de carbone, le chlorure d'éthylène, le tétrachloréthane, le 1,2-dichloréthane, le 1,1,1-trichloréthane, le perchloréthylène, le trichloréthylène, le 1,1,2-trichlorotrifluoroéthane, les dichlorobenzènes, les trichlorobenzènes, le toluène ou les xylènes, de préférence le 1,2-dichlorobenzène, le toluène ou le xylène.

6. Procédé selon l'une quelconque des revendications 1 à 5, caractérisé par le fait qu'on effectue l'extraction selon le stade (a) 3 à 10 fois, de préférence 3 à 7 fois.

7. Procédé selon l'une quelconque des revendications 1 à 6, caractérisé par le fait qu'on effectue l'extraction selon le stade (a) à des températures comprises entre la température ambiante et 95° C.

8. Procédé selon la revendication 7, caractérisé par le fait qu'on extrait à des températures de 45 à 85° C.

9. Procédé selon la revendication 1, caractérisé par le fait qu'on règle la phase aqueuse selon le stade (b) à un pH de 8 à 10, de préférence 9 à 9,5.

10. Procédé selon la revendication 1, caractérisé par le fait qu'on ajoute, à la masse fondue, du polyéthylèneglycol avant la distillation selon le stade (c).

11. Procédé selon la revendication 1, caractérisé par le fait qu'on élimine les parties très volatiles de la masse fondue avant la distillation moléculaire dans un évaporateur à film tombant.

12. Procédé selon la revendication 1, caractérisé par le fait qu'on élimine les parties très volatiles de la masse fondue directement dans l'appareil de distillation moléculaire dans un stade supplémentaire de distillation.

13. Procédé selon la revendication 1, caractérisé par le fait qu'on effectue la distillation moléculaire sous une pression très basse, de préférence de l'ordre de $10^{-3}$ mbar.

14. Procédé selon la revendication 1, caractérisé par le fait qu'on distille la masse fondue selon le stade (c) deux fois dans l'appareil à distillation moléculaire.

15. Procédé selon la revendication 1, caractérisé par le fait que l'introduction de la masse fondue selon le stade (d) dans la dispersion aqueuse de l'éther 4,2',4'-trichloro-2-hydroxydiphényle cristallisé s'effectue à des températures de 10 à 50° C, en particulier de 30 à 45° C.

16. Procédé selon la revendication 1, caractérisé par le fait que la dispersion aqueuse selon le stade (d) contient au moins 0,1, en particulier au moins 0,5 % d'éther 4,2',4'-trichloro-2-hydroxydiphényle cristallisé.

17. Procédé selon la revendication 1, caractérisé par le fait que le stade (d) est effectué dans un broyeur à colloïde du type à rotor-stator.

18. Procédé selon la revendication 1, caractérisé par le fait que, dans le cas où le mélange réactionnel ne contient pas ou seulement que très peu de solvant, le stade (a) est effectué sans entraînement à la vapeur d'eau.